(19) Europäisches Patentamt
European Patent Office
Office européen des brevets

(11) Veröffentlichungsnummer : **0 104 376**
**B1**

(12) # EUROPÄISCHE PATENTSCHRIFT

(45) Veröffentlichungstag der Patentschrift : **06.05.87**

(21) Anmeldenummer : **83107754.0**

(22) Anmeldetag : **06.08.83**

(51) Int. Cl.⁴ : **C 07 C 93/10**, C 07 C101/28, C 07 C119/12, C 07 D295/08// C07F9/50, C07F15/00

(54) **Verfahren zur Herstellung von optisch aktiven Aminen und Enaminen.**

(30) Priorität : **27.08.82 CH 5110/82**
**01.07.83 CH 3642/83**

(43) Veröffentlichungstag der Anmeldung : **04.04.84 Patentblatt 84/14**

(45) Bekanntmachung des Hinweises auf die Patenterteilung : **06.05.87 Patentblatt 87/19**

(84) Benannte Vertragsstaaten :
**AT BE CH DE FR GB IT LI NL**

(56) Entgegenhaltungen :
**EP-A- 0 068 506**
**AMERICAN CHEMICAL SOCIETY, Symposium Series, Band 185, 1982, pages 187-193. K. TANI et al.: "Rhodium(I) catalyzed enantioselective hydrogen migration of prochiral allylamines"**
**TETRAHEDRON LETTERS, Nr. 37, Juli 1968, Seiten 4023-4026, Pergamon Press, Oxford, GB. L. HORNER et al.: "Hydrierung und Isomerisierung von Olefinen mit Homogen gelösten Phosphin-Rhodium-Komplexen"**

(73) Patentinhaber : **F. HOFFMANN-LA ROCHE & CO. Aktiengesellschaft**
**CH-4002 Basel (CH)**

(72) Erfinder : **Hansen, Hans-Jürgen, Prof.**
**Bettingerstrasse 67**
**CH-4125 Riehen (CH)**
Erfinder : **Schmid, Rudolf, Dr.**
**Sempacherstrasse 51**
**CH-4053 Basel (CH)**
Erfinder : **Schmid, Max, Dr.**
**Stapferstrasse 21**
**CH-5200 Brugg (CH)**

(74) Vertreter : **Cottong, Norbert A. et al**
**Grenzacherstrasse 124 Postfach 3255**
**CH-4002 Basel (CH)**

**EP 0 104 376 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von optisch aktiven, in Stellung 4 funktionalisierten Enaminen oder Iminen der allgemeinen Formel

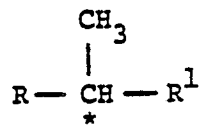

(I)

worin R geschütztes Hydroxymethyl, geschütztes Formyl oder Alkoxycarbonyl und $R^1$ eine Gruppe der Formel

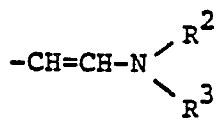

oder $—CH_2—CH{=}NR^2$ darstellen, wobei $R^2$ und $R^3$ niederes Alkyl oder Cycloalkyl oder $R^2$ und $R^3$ zusammen mit dem Stickstoffatom einen heterocyclischen Ring darstellen, welches dadurch gekennzeichnet ist, dass man ein Amin der allgemeinen Formel

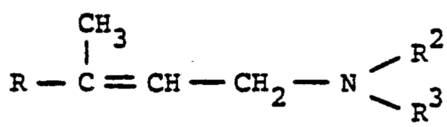

(II)

worin R, $R^2$ und $R^3$ die obige Bedeutung haben und $R^3$ zusätzlich Wasserstoff bedeuten kann, mit Hilfe eines Komplexes eines Metalles der Gruppe VIII und einer optisch aktiven, in (R)- oder (S)-Form vorliegenden Verbindung der allgemeinen Formel

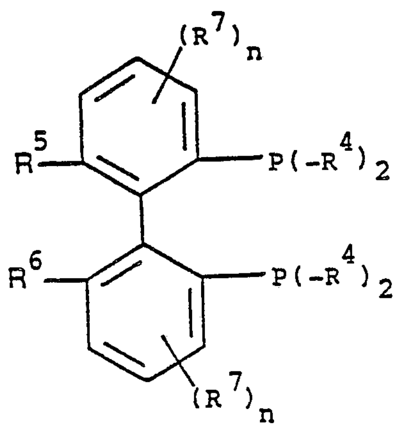

(III)

worin $R^4$ Phenyl, $R^5$ und $R^6$, welche gleich oder verschieden sein können, Wasserstoff, niederes Alkyl, niederes Alkoxy, Di-niederes Alkylamino, geschütztes Hydroxymethyl oder $R^5$ und $R^6$ zusammen die Gruppen $(—CH_2—)_m$, $—CH_2—O—CH_2—$,

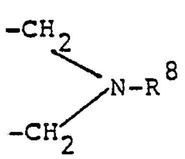

oder

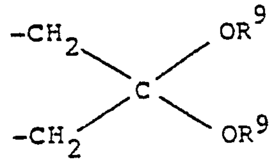

.

bedeuten, wobei m eine Zahl 3 bis 5, $R^8$ niederes Alkyl, Phenyl oder Benzyl und $R^9$ niederes Alkyl oder

beide $R^9$ zusammen Di- oder Trimethylen darstellen, $R^7$ Methyl, niederes Alkoxy, Di-niederes Alkylamino oder Fluor und n die Zahl 0, 1, 2 oder 3 bedeuten, oder einer optisch aktiven, in (R)- oder (S)-Form vorliegenden Verbindung der allgemeinen Formel

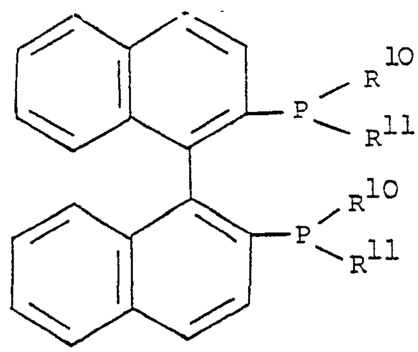

(IV)

worin $R^{10}$ und $R^{11}$ Phenyl oder Cyclohexyl bedeuten und die Naphthalinringe gegebenenfalls noch in ortho-Stellung mit Methyl, Aethyl, Halogen, Di-niederes Alkylamino oder niederes Alkoxy substituiert sind, isomerisiert.

Die Erfindung betrifft ebenfalls die neuen optisch aktiven Enamine und Imine der obigen Formel I, sowie deren Verwendung zur Herstellung von beispielsweise natürlichem Vitamin E, natürlichem Vitamin $K_1$ oder auch von Riechstoffen.

Aus der Zeitschrift « American Chemical Society, Symposium Series, Bd. 195 (1982), S. 187-193 » ist die Isomerisierung von Allylaminen zu Enaminen oder Iminen mittels Rhodium-Phosphin-Komplexen bereits bekannt. Es handelt sich dabei — im Gegensatz zu den erfindungsgemäss verwendeten Allylaminen — jedoch um solche, welche lediglich eine allylische Heterofunktion aufweisen.

Die Verbindungen der Formel I können leicht in an sich bekannter Weise zu den optisch aktiven Aldehyden der allgemeinen Formel

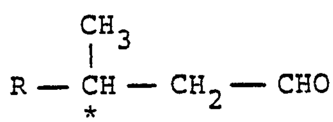

(V)

worin R die obige Bedeutung hat, hydrolysiert werden. Diese Aldehyde, bzw. die davon abgeleiteten Säuren oder Alkohole, sind bekannte Verbindungen oder Analoge bzw. Derivate bekannter Verbindungen, welche in bekannter Weise in natürliches Vitamin E oder natürliches Vitamin $K_1$ übergeführt werden können.

Aus der deutschen Offenlegungsschrift (DOS) No. 27 20 775 und aus der europäischen Patentpublikation No. 39 830 sind bereits Verfahren zur Herstellung von optisch aktiven Verbindungen der Formel V bzw. von Analogen hiervon bekannt. Gemäss obiger DOS werden diese Verbindungen zwar mit hoher optischer Reinheit, jedoch auf mikrobiologischem Wege hergestellt, und gemäss dem Verfahren der erwähnten europäischen Patentpublikation werden diese Verbindungen nur mit einer optischen Reinheit von lediglich ca. 60 % erhalten.

Es bestand somit ein Bedürfnis nach einem chemischen, insbesondere einem katalytischen, Verfahren zur Herstellung der optisch aktiven Verbindungen der Formeln I bzw. V, gemäss welchem diese Verbindungen insbesondere mit einer hohen optischen Reinheit erhalten werden können. Dies ist nun mittels des erfindungsgemässen Verfahrens gelungen, da gemäss diesem Verfahren solche Verbindungen mit einer optischen Reinheit von bis zu 99 % erhalten werden. Dieses neue Verfahren, sowie auch die neuen Verbindungen der Formel I stellen somit eine wertvolle Bereicherung des Standes der Technik dar.

Als Schutzgruppen für die Hydroxymethylgruppen kommen im Rahmen der vorliegenden Erfindung insbesondere in Frage die üblichen, Aether-bildenden Gruppen wie z. B. Benzyl, Methyl, tert.-Butyl, Allyl, Methoxymethyl sowie auch Silylätherbildende Gruppen, wie z. B. Trimethylsilyl, tert. Butyldimethylsilyl.

Der Ausdruck « geschütztes Formyl » bedeutet insbesondere acetalisiertes Formyl. Acetalisierte Formylgruppen sind beispielsweise Reste der Formel

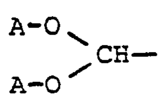

in der A $C_1$-$C_4$-Alkyl bedeutet, oder beide Substituenten A zusammen $C_2$-$C_4$-Alkylen darstellen.

Der Ausdruck « Alkoxycarbonyl » bedeutet insbesondere solche Gruppen, in denen der Alkylrest 1-4 Kohlenstoffatome aufweist, wie Methoxycarbonyl, Aethoxycarbonyl. Der Ausdruck « niederes Alkyl » bedeutet insbesondere geradkettige oder verzweigte Alkylgruppen mit 1-4 Kohlenstoffatomen wie Methyl, Aethyl, Propyl, Isopropyl, n-Butyl, Isobutyl und tert.-Butyl. Der Ausdruck « Cycloalkyl » bedeutet insbesondere Cyclopropyl, Cyclopentyl, Cyclohexyl. Falls die Substituenten $R^2$ und $R^3$ zusammen mit dem Stickstoffatom einen heterocyclischen Ring darstellen, ist dies vorzugsweise ein Pyrrolidin- oder Piperidinring. In einem derartigen Ring können jedoch auch noch ein weiteres Sauerstoffatom oder ein gegebenenfalls alkyliertes ($C_1$-$C_4$) oder benzyliertes Stickstoffatom enthalten sein, z. B. der Morpholin- oder Piperazinring.

Die erwähnten Phenyl- und Benzylreste können, im Rahmen der vorliegenden Erfindung, sowohl unsubstituiert als auch in ortho-, meta- oder para-Stellung oder auch mehrfach substituiert sein. Als Substituenten kommen hier in Frage niedere Alkyl- oder niedere Alkoxygruppen, vorzugsweise Methyl- oder Methoxygruppen, oder auch Di-niederes Alkylamino, vorzugsweise Dimethylaminogruppen, sowie Fluor. Die Ausdrücke « niederes Alkoxy », « Di-niederes Alkylamino » und « niederes Alkoxycarbonyl » bedeuten Gruppen, in denen der Alkylrest die vorhergehend erwähnte Bedeutung haben kann.

Bevorzugte Verbindungen der Formel III sind solche, worin $R^4$ unsubstituiertes oder Methyl oder Fluor substituiertes Phenyl, $R^5$ und $R^6$ gleich sind und niederes Alkyl oder zusammen die Gruppe $—CH_2—O—CH_2—$, n die Zahl 0 oder 1 und $R^7$ Methyl, Fluor oder Di-niederes Alkylamino bedeuten.

Die Isomerisierung einer Verbindung der Formel II zu einer Verbindung der Formel I erfolgt erfindungsgemäss mit Hilfe eines Katalysator-Komplexes aus einem Metall der Gruppe VIII und einem Liganden der allgemeinen Formel III oder IV.

Von den Metallen der Gruppe VIII können als bevorzugt genannt werden Rhodium, Iridium und Cobalt, insbesondere Rhodium.

Die erwähnten Katalysator-Komplexe können in einfacher und an sich bekannter Weise hergestellt werden. Dies erfolgt z. B. indem man eine Verbindung der Formel III oder IV mit einer Verbindung, welche ein Metall der Gruppe VIII abgeben kann, in einem geeigneten inerten Lösungsmittel umsetzt. Dies kann einerseits dadurch geschehen, dass man die Phosphorverbindungen der Formeln III und IV als solche, in einer Lösung einer zu isomerisierenden Verbindung der Formel II, mit einer ein Metall der Gruppe VIII abgebenden Verbindung in Kontakt bringt ; d. h. der Katalysator-Komplex kann in situ gebildet werden. Andererseits können die Phosphorverbindungen der Formeln III und IV zunächst in einem geeigneten, organischen oder wässrigen Lösungsmittel mit einer ein Metall der Gruppe VIII abgebenden Verbindung zu dem entsprechenden Katalysator-Komplex umgesetzt werden und dieser dann zu einer Lösung der zu isomerisierenden Verbindung der Formel II gegeben werden. Die letztere Methode ist bevorzugt. Verbindungen, welche ein Metall der Gruppe VIII abgeben können, sind bekannt. Als geeignete, z. B. Rhodium abgebende Verbindungen können beispielsweise genannt werden Rhodiumtrichlorid-Hydrat, Rhodiumtribromid-Hydrat, Rhodiumsulfat, oder auch organische Rhodiumkomplexe mit Aethylen, Propylen sowie mit bis-Olefinen, z. B. 1,5-Cyclooctadien, 1,5-Hexadien, Bicyclo[2.2.1]hepta-2,5-dien oder mit weiteren Dienen, welche mit Rhodium leicht lösliche Komplexe bilden. Bevorzugte, Rhodium abgebende Verbindungen sind z. B. Di-μ-chloro-bis[$\eta^4$-1,5-cyclooctadien]dirhodium(I) oder auch Di-μ-chloro-bis[$\eta^4$-norbornadien]dirhodium(I).

Als weitere geeignete, eines der anderen Metalle der Gruppe VIII abgebende Verbindungen können beispielsweise noch genannt werden : Iridiumtrichlorid-Hydrat, Di-μ-chloro-bis[$\eta^4$-1,5-cyclooctadien]diiridium(I) ; Cobaltdichlorid, Cobalt(II)-acetat oder -naphthenat, Cobalt(II)- oder Cobalt(III)-acetylacetonat.

Sowohl die Umsetzung der Phosphorverbindungen der Formeln III und IV mit einer ein Metall der Gruppe VIII abgebenden Verbindung, wie auch die erwähnte Isomerisierung können in geeigneten, unter der Reaktionsbedingungen inerten organischen Lösungsmitteln durchgeführt werden. Als solche können insbesondere genannt werden niedere Alkanole wie z. B. Methanol oder Aethanol, aromatische Kohlenwasserstoffe wie Benzol oder Toluol, cyclische Aether wie Tetrahydrofuran oder Dioxan, Ester wie z. B. Essigester oder auch Gemische hiervon. Weiterhin können die Komplexbildungen in wässrigem Medium oder auch in Dichlormethan durchgeführt werden.

Das Verhältnis zwischen Metall der Gruppe VIII und den Liganden der Formeln III und IV liegt zweckmässig zwischen 0,05 und 5 Mol, vorzugsweise zwischen 0,5 und 2 Mol Metall pro Mol Ligand der Formel III oder IV.

Die Menge an Metall in den Komplexen mit den Liganden der Formeln III und IV, bezogen auf die zu isomerisierenden Verbindungen, liegt zweckmässig zwischen 0,01 und 2 Mol%, vorzugsweise zwischen 0,05 und 1 Mol% und insbesondere zwischen 0,1 und 0,5 Mol%.

Die Isomerisierung kann zweckmässig in einem inerten, organischen Lösungsmittel und bei einer Temperatur von Raumtemperatur bis 130 °C durchgeführt werden. Diese Reaktion erfolgt vorzugsweise bei erhöhter Temperatur, d. h. je nach verwendetem Lösungsmittel entweder bei Rückflusstemperatur des Reaktionsgemisches oder in einem geschlossenen Gefäss, unter Druck.

Die zu isomerisierenden Verbindungen der allgemeinen Formel II sind bekannte Verbindungen oder Derivate oder Analoge bekannter Verbindungen, welche leicht in an sich bekannter Weise hergestellt werden können. Sie können als (E/Z)-Gemische vorliegen, vorzugsweise jedoch in der reinen (E)- oder (Z)-Form.

Die optisch aktiven Verbindungen der Formel III können z. B. dadurch hergestellt werden, dass man

eine racemische Verbindung der allgemeinen Formel

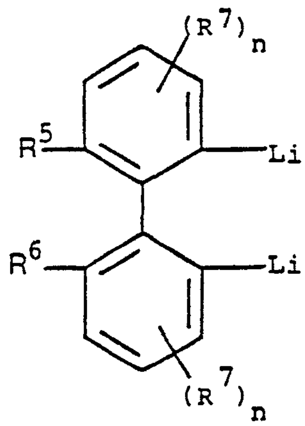

(VI)

worin $R^5$, $R^6$, $R^7$ und n die obige Bedeutung haben, mit einer Verbindung der allgemeinen Formel

$$X—P(—R^4)_2 \quad \text{(VII)}$$

worin $R^4$ die obige Bedeutung hat und X eine austretende Gruppe darstellt, umsetzt und eine erhaltene racemische Verbindung der Formel III in die optischen Antipoden auftrennt.

Der Ausdruck « austretende Gruppe » bedeutet in diesem Zusammenhang insbesondere Gruppen wie z. B. Halogen, insbesondere Chlor oder Brom, sowie Alkoxygruppen wie Methoxy.

Die Umsetzung einer Verbindung der Formel VI mit einer Verbindung der Formel VII kann in an sich bekannter Weise erfolgen. Die Umsetzung erfolgt zweckmässig in einem inerten, aprotischen organischen Lösungsmittel, wie z. B. einem Aether wie etwa Diäthyläther, Tetrahydrofuran, Dimethoxyäthan. Die Reaktion erfolgt auch zweckmässig bei einer Temperatur von Raumtemperatur bis — 120 °C, vorzugsweise unterhalb — 60 °C und insbesondere unterhalb — 90 °C. Der Druck ist hierbei keine kritische Grösse und die Reaktion kann ohne weiteres bei Atmosphärendruck erfolgen.

Die Spaltung einer racemischen Verbindung der Formel III in die optischen Antipoden kann in an sich bekannter Weise durchgeführt werden. Vorzugsweise erfolgt dies z. B. durch Komplexbildung mit Di-μ-chloro-bis[(R)-2-[1-(dimethylamino)äthyl]phenyl-C,N]-dipalladium(II), Trennung der beiden diastereomeren Komplexe durch fraktionierte Kristallisation und anschliessende reduktive Freisetzung der entsprechenden Antipoden.

Die racemischen Verbindungen der Formel VI, sowie die Verbindung der Formel VII sind bekannte Verbindungen oder Analoge bekannter Verbindungen, welche leicht in an sich bekannter Weise hergestellt werden können.

## Beispiel 1

A) In einem Pyrex-Bombenrohr von ca. 25 ml Inhalt wurden 2,3 g (10,8 mMol) (E)-N,N-Diäthyl-4-tert.-butoxy-3-methyl-2-butenylamin, 10 ml trockenes Tetrahydrofuran und 49 mg (0,059 mMol ≙ 0,55 Mol%) [$\eta^4$-Bicyclo[2.2.1]hepta-2,5-dien][(R)-(6,6'-Dimethyl-2,2'-biphenylylen)-bis(diphenylphosphin)]-rhodium(I)-tetrafluoroborat vorgelegt. Nach Entgasung der Mischung wurde das Rohr zugeschmolzen und während 24 Stunden in einem Bombenrohrofen bei 110 °C erhitzt. Die dunkelbraune Reaktionsmischung wurde eingedampft und der braune, ölige Rückstand wurde im Kugelrohr bei ca. 150 °C/15 Torr ($1,95 \cdot 10^3$ Pa) destilliert. Das erhaltene leicht gelbliche Destillat (1,80 g) enthielt gemäss Gaschromatographie 68 % (1E,3R)-N,N-Diäthyl-4-tert.-butoxy-3-methyl-1-butenylamin.

$^1$H-NMR (60 MHz, $CDCl_3$) : 5,9 ppm (d, $J_{trans}$ = 14 Hz, H—C(1)) ; 4,05 ppm (dxd, $J_{trans}$ = 14 Hz, J(2,3) = 7 Hz, H—C(2)).

B) Das vorhergehend erhaltene Destillat wurde in 13,5 ml Tetrahydrofuran aufgenommen, mit 6,5 ml 0,5 N HCl und 2 ml Essigsäure versetzt. Die homogene Mischung wurde 15 Minuten bei 0 °C gerührt. Zur Aufarbeitung wurde das Ganze in Wasser gegossen und dreimal mit Aether extrahiert. Die vereinigten organischen Auszüge wurden zweimal mit 1 NHCl, dann mit verdünnter $NaHCO_3$-Lösung und gesättigter NaCl-Lösung gewaschen, über $Na_2SO_4$ getrocknet, filtriert und eingedampft. Nach Kugelrohrdestillation des erhaltenen Oeles bei ca. 100 °C/15 Torr ($1,95 \cdot 10^3$ Pa) erhielt man 1,03 g (R)-4-tert.-Butoxy-3-methylbutanal als farbloses Oel mit einer gaschromatographischen Reinheit von 97 % ; $[\alpha]_D^{20} = + 23°$ (c = 4,98 % in $CHCl_3$). Durch chemische Korrelation mit (R)-3-Methyl-γ-butyrolacton wurde die absolute Konfiguration bestimmt und eine optische Reinheit von etwa 93 % abgeschätzt.

In zum Vorhergehenden analoger Weise wurde bei Verwendung von [$\eta^4$-Bicyclo[2.2.1]hepta-2,5-dien][(S)-(6,6'-Dimethyl-2,2'-biphenylylen)-bis(diphenylphosphin)]rhodium(I)-tetrafluoroborat als Katalysator das (1E,3S)-N,N-Diäthyl-4-tert.-butoxy-3-methyl-1-butenylamin erhalten ; GC-Reinheit 79 % ; $[\alpha]_D^{20}$ —4,65° (c = 5 % in $CHCl_3$). Das entsprechende (S)-4-tert.-Butoxy-3-methyl-butanal wurde wie vorhergehend beschrieben hergestellt : Gaschromatographische Reinheit 99 % ; $[\alpha]_D^{20}$ —25,3° (c = 4,9 % in $CHCl_3$).

Die enantiomere Reinheit (ee) wurde in Analogie zur Methode von D. Valentine, Jr. et al., in J. Org. Chem. 41, 62 (1976) durch Oxidation zur Säure, Amidbildung mit (R)-α-Methyl-4-nitrobenzylamin und Analyse der diastereomeren Amide mittels GC bzw. LC bestimmt. Dabei wurde ein SR/RR Diastereomerenverhältnis von 96,3 : 3,7 gefunden, woraus sich für die enantiomere Reinheit ein Wert von 92,6 % ableitet.

Beispiel 2

Das gemäss Beispiel 1 verwendete [$\eta^4$-Bicyclo[2.2.1]hepta-2,5-dien][(R)-(6,6'-Dimethyl-2,2'-biphenylylen)-bis(diphenylphosphin)]rhodium(I)-tetrafluoroborat wurde wie folgt hergestellt.

A) In einem 100 ml Sulfierkolben, versehen mit Tropftrichter, Thermometer, Gummiseptum und Magnetrührer wurden unter Argonbegasung 680 mg (2 mMol) (RS)-2,2'-Dibrom-6,6'-dimethyl-biphenyl vorgelegt und 20 ml absoluter Diäthyläther mittels einer Spritze injiziert. Die entstandene Lösung wurde auf ca. —90° bis —100 °C gekühlt und bei dieser Temperatur tropfenweise mit 5,6 ml einer ca. 1,4 M tert.-Butyllithiumlösung in Pentan (8 mMol) versetzt. Die Reaktionslösung wurde noch 20 Minuten bei ca. —100 °C nachgerührt, wobei sie sich langsam weisslich trübte. Dann wurde eine Lösung von 880 mg (4 mMol) Chlordiphenylphosphin in 5 ml abs. Diäthyläther innerhalb 15 Minuten bei ca. —90° bis —100 °C eingetropft. Die Reaktionsmischung wurde während 2 Stunden auf Raumtemperatur erwärmen gelassen, wobei bei ca. —60 °C die Ausfällung eines weissen Niederschlages einsetzte, und noch 1 Stunde bei Raumtemperatur nachgerührt. Zur Aufarbeitung wurde das Gemisch unter Argon mit Wasser und Dichlormethan versetzt, die organische Phase abgetrennt, mit Wasser gewaschen und durch Filtration über $Na_2SO_4$ getrocknet. Der nach dem Eindampfen erhaltene kristalline Rückstand wurde aus Essigsäureäthylester umkristallisiert und man erhielt 620 mg (RS)-(6,6'-Dimethyl-2,2'-biphenylylen)-bis-(diphenylphosphin) als weisse Kristalle mit einem Schmelzpunkt von 242-243 °C. Aus der Mutterlauge wurden nach Kristallisation aus Aethanol/Toluol weitere 84 mg Produkt mit einem Schmelzpunkt von 240-242 °C gewonnen ; Gesamtausbeute 704 mg.

B) Das vorhergehend verwendete (RS)-2,2'-Dibrom-6,6'-dimethyl-biphenyl wurde wie folgt hergestellt :

Zu einer Lösung von 4,24 g (20 mMol) (RS)-6,6'-Dimethyl-2,2'-biphenyldiamin in 12 ml 48 %iger wässriger Bromwasserstofflösung wurde bei —5° bis 0 °C eine Lösung von 2,77 g (40,1 mMol) Natriumnitrit in 5 ml Wasser innerhalb von 2 Stunden zugetropft. Die eiskalte, dunkle Diazoniumsalzlösung wurde in einen Tropftrichter transferiert und innerhalb von 15 Minuten zu einer heissen (70-75 °C) Lösung von 25 ml einer 2M Kupfer (I)bromid-Lösung in 48 %iger wässriger Bromwasserstofflösung getropft. Die Diazoniumsalzlösung wurde dabei durch gelegentliches Zufügen von Eis bei 0 °C gehalten. Nach beendigtem Zutropfen wurde noch 5 Minuten am Rückfluss gekocht. Die abgekühlte Reaktionslösung wurde dann dreimal mit je 100 ml Aether extrahiert. Die vereinigten organischen Extrakte wurden zweimal mit je 50 ml 2 N HCl, zweimal mit je 50 ml gesättigter $NaHCO_3$-Lösung und dreimal mit je 50 ml Wasser gewaschen und dann über $Na_2SO_4$ getrocknet. Das nach Filtration und Eindampfen erhaltene Produkt (6 g) bestand gemäss Gaschromatographie aus einem 8 : 23 : 59-Gemisch von 2-Brom-6,6'-dimethyl-biphenyl, 2-Brom-2'-hydroxy-6,6'-dimethyl-biphenyl und (RS)-2,2'-Dibrom-6,6'-dimethyl-biphenyl. Chromatographie an Kieselgel (Hexan/Aether 9 : 1) lieferte 3,5 g eines 12 : 87 Gemisches von 2-Brom-6,6'-dimethyl-biphenyl und (RS)-2,2'-Dibrom-6,6'-dimethyl-biphenyl, aus welchem durch zweimaliges Umkristallisieren aus Pentan schliesslich 1,3 g reines (RS)-2,2'-Dibrom-6,6'-dimethyl-biphenyl erhalten wurden mit einem Schmelzpunkt von 111°-112 °C.

C) Eine Suspension von 2,49 g (4,52 mMol) (RS)-(6,6'-Dimethyl-2,2'-biphenylylen)-bis(diphenylphosphin) und 1,31 g (2,26 mMol) Di-μ-chloro-bis[(R)-2-(1-(dimethylamino)äthyl)phenyl-C,N]-dipalladium(II) in 100 ml Methanol wurde unter Argon bei Raumtemperatur gerührt, bis eine homogene Lösung entstanden war (ca. 4 Stunden). Dazu wurde eine Lösung von 0,95 g (9,04 mMol) Ammoniumtetrafluoroborat in 63 ml Wasser getropft, wobei nach Zugabe von ca. 20 ml die Ausfällung eines gelblichen Niederschlages einsetzte. Der entstandene Niederschlag wurde abfiltriert, mit Methanol/Wasser 1 : 1 gewaschen und bei 0,2 Torr (26 Pa), über Phosphorpentoxid getrocknet, worauf 1,79 g gelbliche Kristalle erhalten wurden. Das Filtrat wurde mit 50 ml Wasser verdünnt und bis zur Koagulation der entstandenen Fällung gerührt (1 Stunde). Filtration und Trocknung lieferten weitere 1,84 g gelbliche Kristalle ; Gesamtausbeute 3,63 g.

Die vereinigten Kristallfraktionen wurden in 150 ml Dichlormethan/Diäthyläther 1 : 2 gelöst. Durch langsames Zutropfen von 50 ml Hexan wurde ein Niederschlag erhalten, wobei nach Filtration 1,5 g gelbliche Kristalle erhalten wurden. Dieses Material wurde noch zweimal aus 10 ml Dichlormethan durch

portionenweises Zugeben von viermal 10 ml Diäthyläther umkristallisiert. Auf diese Weise wurden 1,07 g [(R)-2-(1-(Dimethylamino)äthyl)phenyl-C,N][(R)-(6,6'-dimethyl-2,2'-biphenylylen)-bis(diphenylphosphin)]palladium(II)-tetrafluoroborat mit einem Schmelzpunkt von 213-216 °C erhalten ; $[\alpha]_D^{20}$ = + 301,4° (c = 1 % in $CHCl_3$).

In eine Suspension von 42,6 mg (1,12 mMol) Lithiumaluminiumhydrid in 15 ml abs. Tetrahydrofuran wurden bei Raumtemperatur 1 g (1,12 mMol) des vorhergehend hergestellten Tetrafluoroborates in kleinen Portionen innerhalb von 30 Minuten eingetragen. Die entstehende schwarze Reaktionsmischung wurde 1 Stunde nachgerührt. Danach wurde die Reaktion durch Zugabe von einigen Tropfen gesättigter NaCl-Lösung abgebrochen, das Gemisch mit Aktivkohle versetzt und über ein Bett von $Na_2SO_4$ und Celite filtriert, und mit viermal 10 ml Tetrahydrofuran nachgewaschen. Der nach dem Eindampfen des Filtrates anfallende schwarz-braune Rückstand wurde in wenig Dichlormethan aufgenommen und über eine kurze Säule von Kieselgel mit Dichlormethan filtriert. Das gelbe Filtrat wurde eingedampft und der kristalline Rückstand wurde aus Aethanol/Toluol umkristallisiert. Man erhielt 121 mg (R)-(6,6'-Dimethyl-2,2'-biphenylylen)-bis(diphenylphosphin) als weisse Kristalle mit einem Schmelzpunkt von 210-212,5 °C ; $[\alpha]_D^{20}$ = — 42,7° (c = 1 % in $CHCl_3$).

D) In einem Schlenkrohr wurden 275 mg (0,5 mMol) (R)-(6,6'-Dimethyl-2,2'-biphenylylen)-bis(diphenylphosphin) und 115 mg (0,25 mMol) Di-μ-chloro-bis[$\eta^4$-bicyclo[2.2.1]hepta-2,5-dien]-dirhodium(I) unter Argon vorgelegt und mit 4 ml deoxygeniertem Methanol versetzt. Die Reaktionsmischung wurde gerührt, bis eine homogene rote Lösung entstanden war (1,5 Stunden). Dazu wurde nun innerhalb von 45 Minuten eine Lösung von 61 mg (0,55 mMol) Natriumtetrafluoroborat in 1,1 ml deoxygeniertem Wasser getropft, wobei ein oranger Niederschlag ausfiel. Nach einer zusätzlichen Rührdauer von 1 Stunde wurde unter Argon filtriert, der Filterrückstand zweimal mit je 0,5 ml Wasser gewaschen und am Hochvakuum getrocknet. Es wurden 360 mg [$\eta^4$-Bicyclo[2.2.1]hepta-2,5-dien][(R)-(6,6'-dimethyl-2,2'-biphenylylen)-bis(diphenylphosphin)]rhodium(I)-tetrafluoroborat erhalten als orange Mikrokristalle mit $[\alpha]_D^{20}$ = — 35,9° (c = 0,445 % in $CHCl_3$).

In analoger Weise wurde das [$\eta^4$-Bicyclo[2.2.1]hepta-2,5-dien][(S)-(6,6'-dimethyl-2,2'-biphenylylen)-bis-(diphenylphosphin)]rhodium(I)-tetrafluoroborat hergestellt ; $[\alpha]_D^{20}$ — 34° (c = 0,54 % in $CHCl_3$).

## Beispiel 3

Ein Pyrex-Bombenrohr wurde unter Stickstoff mit 1,24 g (5,0 mMol) (E)-N,N-Diäthyl-4-benzyloxy-3-methyl-2-butenylamin, 10 ml Tetrahydrofuran (dest. über Natrium/Benzophenon) und 49,8 mg (0,058 mMol) [$\eta^4$-1,5-Cyclooctadien][(S)-(6,6'-dimethyl-2,2'-biphenylylen)-bis(diphenylphosphin)]rhodium(I)-perchlorat beschickt. Nach Entgasung wurde das Rohr zugeschmolzen und in einem Bombenrohrofen während 58 Stunden auf 85 °C erhitzt. Die dunkelbraune Reaktionsmischung wurde eingedampft und der Rückstand im Kugelrohr bei ca. 150 °C/0,2 mmHg destilliert. Dabei wurden 1,05 g eines gelben Oeles erhalten, das gemäss GC- und NMR-Analyse 73 % (1E,3S)-N,N-Diäthyl-4-benzyloxy-3-methyl-1-butenylamin enthielt ; $[\alpha]_D^{20}$ = — 5,1° (c = 1,1 % in Hexan).

Zur Hydrolyse des Enamins wurden 0,85 g des Destillates in 6 ml 50 % Essigsäure aufgenommen, die Mischung 10 Minuten kräftig durchgerührt und dann mit 10 ml Hexan überschichtet und weitere 30 Minuten nachgerührt. Nach Trennung der Phasen wurde die wässrige Lösung noch zweimal mit Aether extrahiert. Die vereinigten organischen Phasen wurden gewaschen (0,2 N HCl, ges. $NaHCO_3$-Lösung, ges. NaCl-Lösung), getrocknet ($Na_2SO_4$), filtriert und eingedampft, und der Rückstand wurde im Kugelrohr bei ca. 120 °C/0,1 mmHg (13 Pa) destilliert, wobei 0,43 g (54 %) (S)-4-Benzyloxy-3-methylbutanal erhalten wurden ; GC-Reinheit ≥ 98 % ; $[\alpha]_D^{20}$ = — 12,0° (c = 4,06 % in $CHCl_3$).

Die Bestimmung der enantiomeren Reinheit erfolgte wie in Beispiel 1 erwähnt in Analogie zur Methode von D. Valentine, Jr. und betrug 98,8 % ee.

Eine in zum vorhergehenden analoger Weise, jedoch bei 75 °C durchgeführte Umsetzung von (E)-N,N-Diäthyl-4-benzyloxy-3-methyl-2-butenylamin mit [$\eta^4$-1,5-Cyclooctadien][(R)-(6,6'-dimethyl-2,2'-biphenylylen)-bis(diphenylphosphin)]rhodium(I)-perchlorat lieferte (R)-4-Benzyloxy-3-methylbutanal mit einer enantiomeren Reinheit von 99,2 % ee ; $[\alpha]_D^{20}$ + 12,05° (c = 4,4 % in $CHCl_3$).

## Beispiel 4

In zu Beispiel 3 analoger Weise (Temperatur 75 °C) wurden folgende Verbindungen hergestellt unter Verwendung von [$\eta^4$-1,5-Cyclooctadien][(R)-(6,6'-dimethyl-2,2'-biphenylylen)-bis(diphenylphosphin)]rhodium(I)-perchlorat als Isomerisierungskatalysator :

Aus (E)-4-Benzyloxy-N,N-dibutyl-3-methyl-2-butenylamin, das

(1E,3R)-4-Benzyloxy-N,N-dibutyl-3-methyl-1-butenylamin : GC-Reinheit 83 % ; $[\alpha]_D^{20}$ + 6,1° (c = 2,13 % in Hexan). Das entsprechende (R)-4-Benzyloxy-3-methylbutanal hat eine enantiomere Reinheit von 98,9 % ee und eine Drehung von $[\alpha]_D^{20}$ + 10,2° (c = 2,7 % in $CHCl_3$) ; GC-Reinheit 86 %.

Aus 1-((E)-4-Benzyloxy-3-methyl-2-butenyl)-piperidin, das

1-((1E,3R)-4-Benzyloxy-3-methyl-1-butenyl)-piperidin : GC-Reinheit 61 % ; $[\alpha]_D^{20}$ + 6,9° (c = 2,15 in Hexan). Das entsprechende (R)-4-Benzyloxy-3-methylbutanal hat eine enantiomere Reinheit von 99,5 % ee und eine Drehung von $[\alpha]_D^{20}$ + 12,14° (c = 3,6 % in $CHCl_3$) ; GC-Reinheit 95 %.

Aus 4-((E)-4-Benzyloxy-3-methyl-2-butenyl)-morpholin, das

4-((1'E,3'R)-4-Benzyloxy-3-methyl-1-butenyl)-morpholin : GC-Reinheit 85 % ; $[\alpha]_D^{20}$ + 6,86° (c = 2,15 % in Hexan). Das entsprechende (R)-4-Benzyloxy-3-methylbutanal hat eine enantiomere Reinheit von 93,1 % ee und eine Drehung von $[\alpha]_D^{20}$ + 11,4° (c = 3,63 % in $CHCl_3$) ; GC-Reinheit 95 %.

## Beispiel 5

In zu Beispiel 3 analoger Weise (Reaktionstemperatur 80 °C) wurde aus (E)-N,N-Diäthyl-3-methyl-4-trimethylsiloxy-2-butenylamin, das (1E,3R)-N,N-Diäthyl-3-methyl-4-trimethylsiloxy-1-butenylamin erhalten in Form eines Oeles. $[\alpha]_D^{20}$ + 17,1° (c = 2,44 % in Hexan) ; GC-Reinheit 50 %.

Zur Bestimmung der optischen Reinheit wurden 0,53 g dieses Oeles in 5 ml 0,5 N HCl und 2 ml Essigsäure aufgenommen. Der Mischung wurden innerhalb von 1 Stunde unter gutem Rühren ca. 100 mg Natriumcyanoborhydrid in Portionen von ca. 20 mg zugefügt. Nach einer zusätzlichen Rührdauer von 2 Stunden wurde die Reaktionsmischung mit 4N Natronlauge alkalisch gestellt, mit $K_2CO_3$ gesättigt und dann während 18 Stunden kontinuierlich mit Aether extrahiert. Der Rückstand aus dem Aetherextrakt wurde durch Chromatographie an $SiO_2$ (Essigsäure-äthylester) gereinigt. Nach Destillation im Kugelrohr bei ca. 100°/0,02 mmHg (2,6 Pa) wurden 148 mg (50,5 %) (R)(+)-2-Methyl-1,4-butandiol als farbloses Oel erhalten ; $[\alpha]_D^{20}$ = + 14,0° (c = 1,9 % in MeOH) ; GC-Reinheit 99 %.

Für den Antipoden (S)(—)-2-Methyl-1,4-butandiol wurde eine spezifische Drehung von $[\alpha]_D^{20}$ = — 14,5° (c = 0,6 % in MeOH) beschrieben. (H.G.W. Leuenberger et al., Helv. Chim. Acta 62, 455 (1979)). Daraus lässt sich eine optische Reinheit von ca. 96 % für das vom Enamin abgeleitete (R)-(+)-2-Methyl-1,4-butandiol abschätzen.

In zum vorhergehenden analoger Weise wurde aus (E)-N,N-Diäthyl-4-(methoxymethoxy)-3-methyl-2-butenylamin das ((1E,3R)-N,N-Diäthyl-4-(methoxymethoxy)-3-methyl-1-butenylamin erhalten. $[\alpha]_D^{20}$ + 4,6° (c = 2,76 % in Hexan) ; GC-Reinheit ca. 40 %. Für das entsprechende (R)(+)-2-Methyl-1,4-butandiol mit einer Drehung von $[\alpha]_D^{20}$ = + 13,6° (c = 0,99 % in Methanol) und einer GC-Reinheit von 85 % wurde eine optische Reinheit von 94 % abgeschätzt.

## Beispiel 6

In zu Beispiel 3 analoger Weise wurde aus (E)-4-Allyloxy-N,N-diäthyl-3-methyl-2-butenylamin, das (1E,3R)-N,N-Diäthyl-3-methyl-4-(1-propenyloxy)-1-butenylamin erhalten. $[\alpha]_D^{20}$ + 17,3° (c = 1,83 % in Hexan).

## Beispiel 7

Ein Pyrex-Bombenrohr wurde mit 2,02 g (10 mMol) (E)-N,N-Diäthyl-4,4-dimethoxy-3-methyl-2-butenylamin, 10 ml Tetrahydrofuran und 83,2 mg (0,10 mMol) [$\eta^4$-Bicyclo-[2.2.1]hepta-2,5-dien][(S)-(6,6'-dimethyl-2,2'-biphenylylen)-bis(diphenylphosphin)]rhodium(I)-tetrafluoroborat beschickt. Nach Entgasung der Mischung wurde das Rohr zugeschmolzen und während 56 Stunden in einem Bombenrohrofen bei 90 °C erhitzt. Die rotbraune Reaktionslösung wurde eingedampft und der Rückstand wurde im Kugelrohr bei ca. 70°/0,2 mmHg (26 Pa) destilliert. Man erhielt (1E,3S)-N,N-Diäthyl-4,4-dimethoxy-3-methyl-1-butenylamin. GC-Reinheit 70 % ; $[\alpha]_D^{20}$ = — 12,9° (c = 4,05 % in Hexan). Das entsprechende (S)-4,4-Dimethoxy-3-methylbutanal hat eine Drehung von $[\alpha]_D^{20}$ — 23,3° (c = 4,66 in Hexan). GC-Reinheit 96 %.

Die enantiomere Reinheit dieses Aldehydes wurde mit Hilfe von NMR-Spektroskopie unter Verwendung des Verschiebungsreagens (Tris[3-(heptafluoropropylhydroxymethyliden)-d-camphorato]-europium(III)) zu ⩾ 90 % ee bei einer Nachweisgrenze von 5 % abgeschätzt.

Das als Ausgangsmaterial verwendete (E)-N,N-Diäthyl-4,4-dimethoxy-3-methyl-2-butenylamin wurde wie folgt hergestellt :

Ein Gemisch von 56,4 g (0,30 Mol) 4-Acetoxy-2-methyl-2-butenal-dimethylacetal, 150 ml Diäthylamin, 675 mg (3 mMol) Palladium(II)-acetat und 3,9 g (15 mMol) Triphenylphosphin wurde unter Argon während 15 Stunden unter Rückfluss gekocht. Nach Einengen des Reaktionsmischung wurde der Rückstand in 170 ml Aether/Pentan 1 : 1 aufgeschlämmt, die Lösung mit etwas Aktivkohle behandelt, über Hyflo filtriert und eingeengt. Das zurückbleibende Oel wurde zunächst bei 40-55°/0,3 mmHg (39 Pa) über eine 5 cm Vigreux-Kolonne destilliert, wobei 57,1 g N,N-Diäthyl-4,4-dimethoxy-3-methyl-2-butenylamin als 90 : 10 (E/Z)-Gemisch erhalten wurden. Dieses Gemisch wurde alsdann über eine 30 cm Füllkörper-Kolonne fraktioniert. Dabei wurden zuerst insgesamt 34,8 g (58 %) Mischfraktionen ((E/Z) = 86 :14) und dann 14,5 g (24 %) reines (E)-N,N-Diäthyl-4,4-dimethoxy-3-methyl-2-butenylamin vom Sdp. 30-32°/0,06 mmHg

gewonnen.

Beispiel 8

Ein Pyrex-Bombenrohr wurde mit 506 mg (2,73 mMol) 4-(Diäthylamino)-2-methyl-2-butensäure-methylester (83 : 17 (E/Z)-Gemisch), 80 mg (0,092 9 mMol) [$\eta^4$-1,5-Cyclooctadien][(R)-(6,6'-dimethyl-2,2'-biphenylylen)-bis(diphenylphosphin)]rhodium(I)-perchlorat und 8 ml Tetrahydrofuran beschickt, entgast, zugeschmolzen und im Bombenrohrofen während 64 Stunden auf 80 °C erhitzt. Die braunschwarze Reaktionsmischung wurde eingedampft und der Rückstand im Kugelrohr bei ca. 150°/15 mmHg ($1{,}95 \cdot 10^3$ Pa) destilliert. Dabei wurden 313 mg eines farblosen Oeles erhalten, das gemäss GC- und NMR-Analyse 53 % (2R,3E)-4-(Diäthylamino)-2-methyl-3-butensäure-methylester sowie 32 % 4-(Diäthylamino)-2-methyl-buttersäure-methylester enthielt ; $[\alpha]_D^{20}$ = — 24,2° (c = 2,35° in Hexan).

Das als Ausgangsmaterial verwendete 4-(Diäthylamino)-2-methyl-2-butensäure-methylester wurde in zu Beispiel 7 analoger Weise hergestellt.

Beispiel 9

Die gemäss den Beispielen 3-6 und 8 verwendeten Isomerisierungskomplexe wurden wie folgt hergestellt :

In einem 50 ml Schlenkrohr wurden 604 mg (1,226 mMol) Di-$\mu$-chloro-bis[$\eta^4$-1,5-cyclooctadien]dirhodium(I) unter Argon vorgelegt und in 20 ml absolutem Tetrahydrofuran aufgelöst. Dazu wurde unter Rühren eine Lösung von 552,5 mg (2,452 mMol) Silberperchlorat-monohydrat in 5 ml absolutem Tetrahydrofuran aus einer Injektionsspritze innerhalb von 10 Minuten getropft. Nach einer zusätzlichen Rührdauer von 1 Stunde wurde das gefällte Silberchlorid unter Argon abfiltriert. Das trübe Filtrat wurde stehen gelassen, bis sich das restliche, durch die Fritte gelaufene kolloidale Silberchlorid abgesetzt hatte. Dann wurde die überstehende Lösung mittels einer Injektionsspritze abdekantiert und in ein Schlenkrohr transferiert. Zu der klaren gelben Lösung wurde dann unter Rühren bei Raumtemperatur eine Lösung von 1,35 g (2,452 mMol) (R)-(6,6'-Dimethyl-2,2'-biphenylylen)-bis(diphenylphosphin) in 30 ml absolutem Tetrahydrofuran mittels einer motorgetriebenen Spritze innerhalb von 2 Stunden getropft, wobei ein oranger Niederschlag entstand. Die Reaktionsmischung wurde 1,5 Stunden nachgerührt, dann unter Argon filtriert, und der Filterrückstand wurde mit 2 ml Tetrahydrofuran gewaschen und am Hochvakuum kurz getrocknet. Auf diese Weise wurden 1,538 g [$\eta^4$-1,5-Cyclooctadien][(R)-(6,6'-dimethyl-2,2'-biphenylylen)-bis(diphenylphosphin)]rhodium(I)-perchlorat als oranges Pulver erhalten. Bei Drehwertbestimmungen in $CH_2Cl_2$ wie auch in Acetonitril wurde Mutarotation beobachtet.

In zum vorhergehenden analoger Weise wurden 1,101 g (2 mMol) (S)-(6,6'-Dimethyl-2,2'-biphenylylen)-bis(diphenylphosphin) umgesetzt. Es wurden 1,50 g [$\eta^4$-1,5-Cyclooctadien][(S)-(6,6'-dimethyl-2,2'-biphenylylen)-bis(diphenylphosphin)]rhodium(I)-perchlorat als oranges Pulver erhalten. Bei der Drehwertbestimmung in $CHCl_3$ wurde Mutarotation beobachtet. $[\alpha]_D^{20}$ (ca. 1 min) + 20,5° ; $[\alpha]_D^{20}$ (5 min). — 26,9° (c = 0,464 % in $CHCl_3$).

Das als Ausgangsmaterial verwendete (R)- bzw. (S)-(6,6'-Dimethyl-2,2'-biphenlylen)-bis(diphenylphosphin) wurde gemäss Beispiel 2 bzw. in Analogie zu Beispiel 2 hergestellt.

**Patentansprüche** (für die Vertragsstaaten : BE, CH, DE, FR, GB, IT, LI, NL)

1. Verfahren zur Herstellung von optisch aktiven, bifunktionellen Verbindungen der allgemeinen Formel

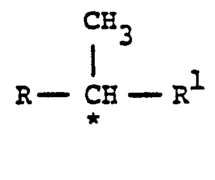

(I)

worin R geschütztes Hydroxymethyl, geschütztes Formyl oder Alkoxycarbonyl und $R^1$ eine Gruppe der Formel

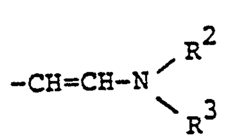

oder $—CH_2—CH=NR^2$ darstellen, wobei $R^2$ und $R^3$ niederes Alkyl oder Cycloalkyl oder $R^2$ und $R^3$ zusammen mit dem Stickstoffatom einen heterocyclischen Ring darstellen, dadurch gekennzeichnet, dass man ein Amin der allgemeinen Formel

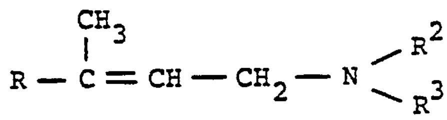

(II)

worin R, $R^2$ und $R^3$ die obige Bedeutung haben und $R^3$ zusätzlich Wasserstoff bedeuten kann, mit Hilfe eines Komplexes eines Metalles der Gruppe VIII und einer optisch aktiven, in (R)- oder (S)-Form vorliegenden Verbindung der allgemeinen Formel

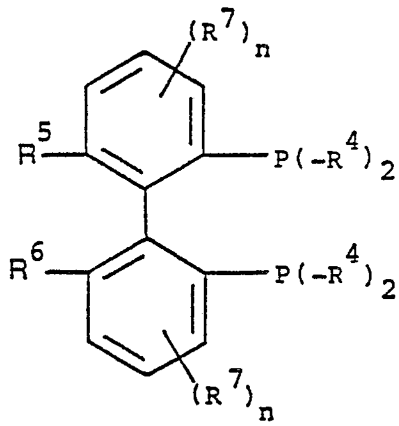

(III)

worin $R^4$ Phenyl, $R^5$ und $R^6$, welche gleich oder verschieden sein können, Wasserstoff, niederes Alkyl, niederes Alkoxy, Di-niederes Alkylamino, geschütztes Hydroxymethyl oder $R^5$ und $R^6$ zusammen die Gruppen $(-CH_2-)_m-CH_2-O-CH_2-$,

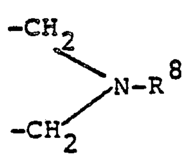

oder

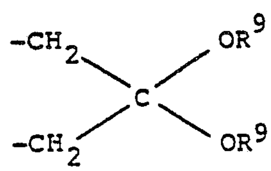

bedeuten, wobei m eine Zahl 3 bis 5, $R^8$ niederes Alkyl, Phenyl oder Benzyl und $R^9$ niederes Alkyl oder beide $R^9$ zusammen Di- oder Trimethylen darstellen, $R^7$ Methyl, niederes Alkoxy, Di-niederes Alkylamino oder Fluor und n die Zahl 0, 1, 2 oder 3 bedeuten, oder einer optisch aktiven, in (R)- oder (S)-Form vorliegenden Verbindung der allgemeinen Formel

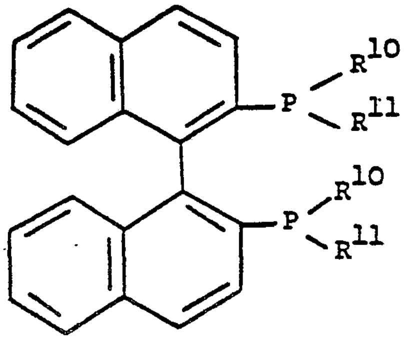

(IV)

worin $R^{10}$ und $R^{11}$ Phenyl oder Cyclohexyl bedeuten und die Naphthalinringe gegebenenfalls noch in ortho-Stellung mit Methyl, Aethyl, Halogen, Di-niederes Alkylamino oder nieder Alkoxy substituiert sind, isomerisiert.

2. Verfahren gemäss Anspruch 1, dadurch gekennzeichnet, dass man die Isomerisierung mittels einer Verbindung der allgemeinen Formel III durchführt.

3. Verfahren gemäss Anspruch 1 oder 2, dadurch gekennzeichnet, dass man als Verbindungen der Formel III solche verwendet, worin $R^4$ unsubstituiertes oder Methyl oder Fluor substituiertes Phenyl, $R^5$ und $R^6$ gleich sind und niederes Alkyl oder zusammen die Gruppe $-CH_2-O-CH_2-$, n die Zahl 0 oder 1 und $R^7$ Methyl, Fluor oder Di-niederes Alkylamino bedeuten.

4. Verfahren gemäss einem der Ansprüche 1 bis 3, dadurch gekennzeichnet, dass man als Verbindung der Formel III (R)- oder (S)-(6,6'-Dimethyl-2,2'-biphenylylen)-bis(diphenylphosphin) verwendet.

5. Verfahren gemäss einem der Ansprüche 1 bis 4, dadurch gekennzeichnet, dass man als Metall der Gruppe VIII Rhodium, Iridium oder Cobalt, vorzugsweise Rhodium, verwendet.

6. Verfahren gemäss einem der Ansprüche 1 bis 5, dadurch gekennzeichnet, dass man den Komplex eines Metalles der Gruppe VIII und einer Verbindung der Formel III oder IV vor der Isomerisierung separat herstellt.

7. Verfahren gemäss einem der Ansprüche 1 bis 6, dadurch gekennzeichnet, dass man als Komplex eines Metalles der Gruppe VIII und einer optisch aktiven, in (R)- oder (S)-Form vorliegenden Verbindung der Formel III das [$\eta^4$-Bicyclo[2.2.1]hepta-2,5-dien][(R)- oder (S)-(6,6'-dimethyl-2,2'-biphenylylen)-bis(diphenylphosphin) rhodium-(I)-tetrafluoroborat verwendet.

8. Verfahren gemäss einem der Ansprüche 1 bis 6, dadurch gekennzeichnet, dass man als Komplex eines Metalles der Gruppe VIII und einer optisch aktiven, in (R)- oder (S)-Form vorliegenden Verbindung der Formel III das [$\eta^4$-1,5-Cyclooctadien][(R)- oder (S)-(6,6'-dimethyl-2,2'-biphenylylen)-bis(diphenylphosphin)]rhodium(I)-perchlorat verwendet.

9. Optisch aktive, bifunktionelle Verbindungen der allgemeinen Formel

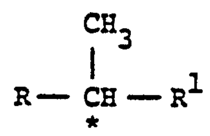

(I)

worin R geschütztes Hydroxymethyl, geschütztes Formyl oder Alkoxycarbonyl und $R^1$ eine Gruppe der Formel

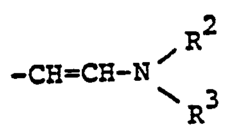

oder $-CH_2-CH=NR^2$ darstellen, wobei $R^2$ und $R^3$ niederes Alkyl oder Cycloalkyl oder $R^2$ und $R^3$ zusammen mit dem Stickstoffatom einen heterocyclischen Ring darstellen.

**Patentansprüche** (für den Vertragsstaat AT)

1. Verfahren zur Herstellung von optisch aktiven, bifunktionellen Verbindungen der allgemeinen Formel

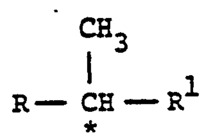

(I)

worin R geschütztes Hydroxymethyl, geschütztes Formyl oder Alkoxycarbonyl und $R^1$ eine Gruppe der Formel

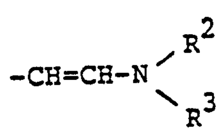

oder $-CH_2-CH=NR^2$ darstellen, wobei $R^2$ und $R^3$ niederes Alkyl oder Cycloalkyl oder $R^2$ und $R^3$ zusammen mit dem Stickstoffatom einen heterocyclischen Ring darstellen, dadurch gekennzeichnet, dass man ein Amin der allgemeinen Formel

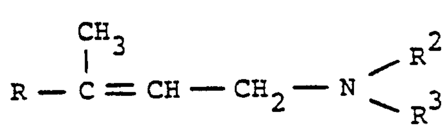

(II)

worin R, $R^2$ und $R^3$ die obige Bedeutung haben und $R^3$ zusätzlich Wasserstoff bedeuten kann, mit Hilfe eines Komplexes eines Metalles der Gruppe VIII und einer optisch aktiven, in (R)- oder (S)-Form vorliegenden Verbindung der allgemeinen Formel

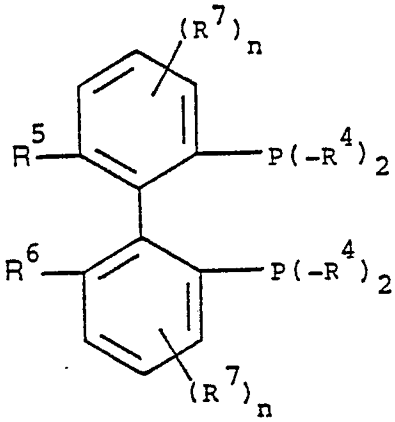

(III)

worin $R^4$ Phenyl, $R^5$ und $R^6$, welche gleich oder verschieden sein können, Wasserstoff, niederes Alkyl, niederes Alkoxy, Di-niederes Alkylamino, geschütztes Hydroxymethyl oder $R^5$ und $R^6$ zusammen die Gruppen $(—CH_2—)_m$, $—CH_2—O—CH_2—$,

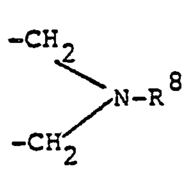

oder

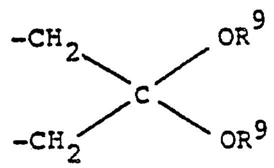

bedeuten, wobei m eine Zahl 3 bis 5, $R^8$ niederes Alkyl, Phenyl oder Benzyl und $R^9$ niederes Alkyl- oder beide $R^9$ zusammen Di- oder Trimethylen darstellen, $R^7$ Methyl, niederes Alkoxy, Di-niederes Alkylamino oder Fluor und n die Zahl 0, 1, 2 oder 3 bedeuten, oder einer optisch aktiven, in (R)- oder (S)-Form vorliegenden Verbindung der allgemeinen Formel

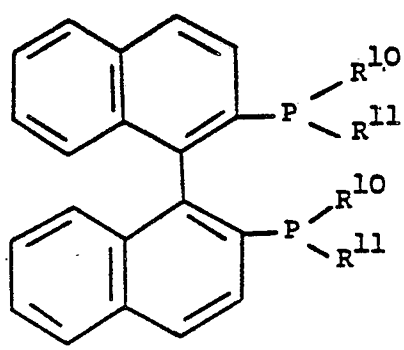

(IV)

worin $R^{10}$ und $R^{11}$ Phenyl oder Cyclohexyl bedeuten und die Naphthalinringe gegebenenfalls noch in ortho-Stellung mit Methyl, Aethyl, Halogen, Di-niederes Alkylamino oder nieder Alkoxy substituiert sind, isomerisiert.

2. Verfahren gemäss Anspruch 1, dadurch gekennzeichnet, dass man die Isomerisierung mittels einer Verbindung der allgemeinen Formel III durchführt.

3. Verfahren gemäss Anspruch 1 oder 2, dadurch gekennzeichnet, dass man als Verbindungen der Formel III solche verwendet, worin $R^4$ unsubstituiertes oder Methyl oder Fluor substituiertes Phenyl, $R^5$ und $R^6$ gleich sind und niederes Alkyl oder zusammen die Gruppe $—CH_2—O—CH_2—$, n die Zahl 0 oder 1 und $R^7$ Methyl, Fluor oder Di-niederes Alkylamino bedeuten.

4. Verfahren gemäss einem der Ansprüche 1 bis 3, dadurch gekennzeichnet, dass man als Verbindung der Formel III (R)- oder (S)-(6,6'-Dimethyl-2,2'-biphenylylen)-bis(diphenylphosphin) verwendet.

5. Verfahren gemäss einem der Ansprüche 1 bis 4, dadurch gekennzeichnet, dass man als Metall der Gruppe VIII Rhodium, Iridium oder Cobalt, vorzugsweise Rhodium, verwendet.

6. Verfahren gemäss einem der Ansprüche 1 bis 5, dadurch gekennzeichnet, dass man den Komplex eines Metalles der Gruppe VIII und einer Verbindung der Formel III oder IV vor der Isomerisierung separat herstellt.

7. Verfahren gemäss einem der Ansprüche 1 bis 6, dadurch gekennzeichnet, dass man als Komplex eines Metalles der Gruppe VIII und einer optisch aktiven, in (R)- oder (S)-Form vorliegenden Verbindung der Formel III das [$\eta^4$-Bicyclo[2.2.1]hepta-2,5-dien][(R)- oder (S)-(6,6'-dimethyl-2,2'-biphenylylen)-bis(diphenylphosphin) rhodium-(I)-tetrafluoroborat verwendet.

8. Verfahren gemäss einem der Ansprüche 1 bis 6, dadurch gekennzeichnet, dass man als Komplex eines Metalles der Gruppe VIII und einer optisch aktiven, in (R)- oder (S)-Form vorliegenden Verbindung der Formel III das [$\eta^4$-1,5-Cyclooctadien][(R)- oder (S)-(6,6'-dimethyl-2,2'-biphenylylen)-bis(diphenylphosphin)] rhodium(I)-perchlorat verwendet.

**Claims** (for the Contracting States : BE, CH, DE, FR, GB, IT, LI, NL)

1. A process for the manufacture of optically active, bifunctional compounds of the general formula

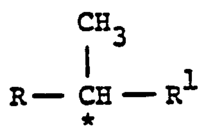
(I)

wherein R represents protected hydroxymethyl, protected formyl or alkoxycarbonyl and $R^1$ represents a group of the formula

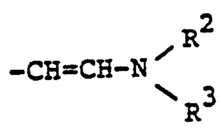

or $—CH_2—CH=NR^2$ in which $R^2$ and $R^3$ represent lower alkyl or cycloalkyl or $R^2$ and $R^3$ together with the nitrogen atom represent a heterocyclic ring, characterized by isomerizing an amine of the general formula

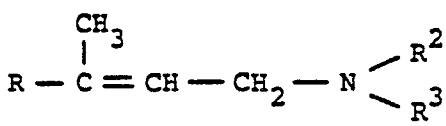
(II)

wherein R, $R^2$ and $R^3$ have the above significance and $R^3$ can additionally signify hydrogen, with the aid of a complex of a metal of Group VIII and an optically active compound of the general formula which is present in the (R)- or (S)-form

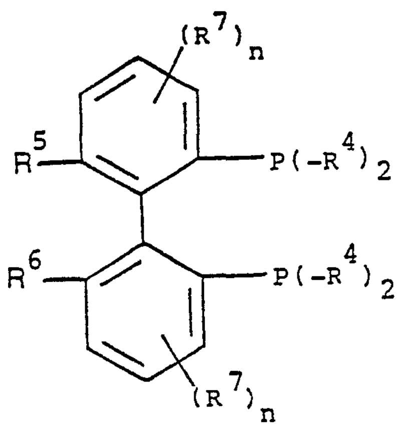
(III)

wherein $R^4$ signifies phenyl, $R^5$ and $R^6$, which can be the same or different, signify hydrogen, lower alkyl, lower alkoxy, di-lower alkylamino, protected hydroxymethyl or $R^5$ and $R^6$ together signify the groups $(—CH_2—)_m$, $—CH_2—O—CH_2—$,

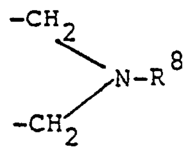 or 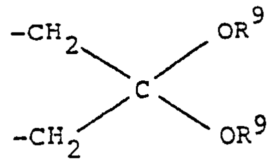

in which m represents a number 3 to 5, $R^8$ represents lower alkyl, phenyl or benzyl and $R^9$ represents lower alkyl or both $R^9$'s together represent di- or trimethylene, $R^7$ signifies methyl, lower alkoxy, di-lower alkylamino or fluorine and n signifies the number 0, 1, 2 or 3, or an optically active compound of the general formula which is present in the (R)- or (S)-form

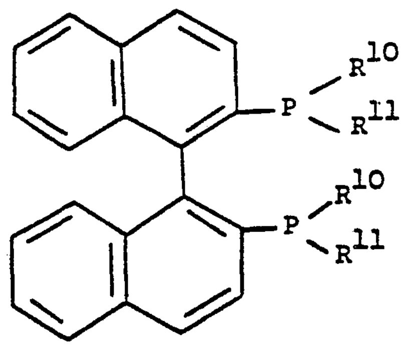

(IV)

wherein $R^{10}$ and $R^{11}$ signify phenyl or cyclohexyl and the naphthalene rings are optionally substituted in the ortho-position with methyl, ethyl, halogen, di-lower alkylamino or lower alkoxy.

2. A process in accordance with claim 1, characterized in that the isomerization is carried out by means of a compound of general formula III.

3. A process in accordance with claim 1 or 2, characterized in that as compounds of formula III there are used those in which $R^4$ signifies unsubstituted phenyl or phenyl substituted with methyl or fluorine, $R^5$ and $R^6$ are the same and signify lower alkyl or together signify the group $-CH_2-O-CH_2-$, n signifies the number 0 or 1 and $R^7$ signifies methyl, fluorine or di-lower alkylamino.

4. A process in accordance with any one of claims 1 to 3, characterized in that (R)- or (S)-(6,6'-dimethyl-2,2'-biphenylylene)-bis (diphenylphosphine) is used as the compound of formula III.

5. A process in accordance with any one of claims 1 to 4, characterized in that rhodium, iridium or cobalt, preferably rhodium, is used as the metal of Group VIII.

6. A process in accordance with any one of claims 1 to 5, characterized in that the complex of a metal of Group VIII and a compound of formula III or IV is prepared separately before the isomerization.

7. A process in accordance with any one of claims 1 to 6, characterized in that [$\eta^4$-bicyclo[2.2.1]hepta-2,5-diene][(R)- or (S)-(6,6'-dimethyl)2,2'-biphenylylene)-bis(diphenylphosphine)rhodium(I) tetrafluoroborate is used as the complex of a metal of Group VIII and an optically active compound of formula III which is present in the (R)- or (S)-form.

8. A process in accordance with any one of claims 1 to 6, characterized in that [$\eta^4$-1,5-cyclooctadiene][(R)- or (S)-(6,6'-dimethyl-2,2'-biphenylylene)-bis(diphenylphosphine)]rhodium(I) perchlorate is used as the complex of a metal of Group VIII and an optically active compound of formula III which is present in the (R)- or (S)-form.

9. Optically active, bifunctional compounds of the general formula

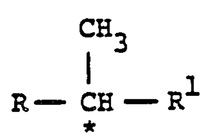

(I)

wherein R represents protected hydroxymethyl, protected formyl or alkoxycarbonyl and $R^1$ represents a group of the formula

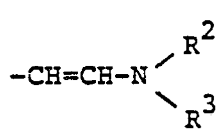

or $-CH_2-CH=NR^2$ in which $R^2$ and $R^3$ represent lower alkyl or cycloalkyl or $R^2$ and $R^3$ together with the

nitrogen atom represent a heterocyclic ring.

**Claims** (for the Contracting State AT)

1. A process for the manufacture of optically active, bifunctional compounds of the general formula

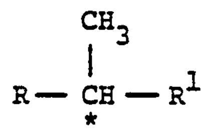

(I)

wherein R represents protected hydroxymethyl, protected formyl or alkoxycarbonyl and $R^1$ represents a group of the formula

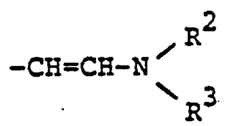

or $—CH_2—CH=NR^2$ in which $R^2$ and $R^3$ represent lower alkyl or cycloalkyl or $R^2$ and $R^3$ together with the nitrogen atom represent a heterocyclic ring, characterized by isomerizing an amine of the general formula

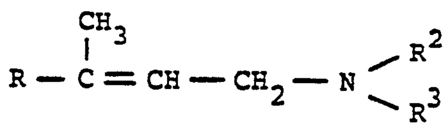

(II)

wherein R, $R^2$ and $R^3$ have the above significance and $R^3$ can additionally signify hydrogen, with the aid of a complex of a metal of Group VIII and an optically active compound of the general formula which is present in the (R)- or (S)-form

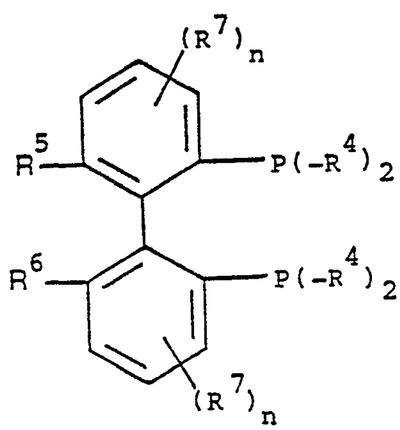

(III)

wherein $R^4$ signifies phenyl, $R^5$ and $R^6$, which can be the same or different, signify hydrogen, lower alkyl, lower alkoxy, di-lower alkylamino, protected hydroxymethyl or $R^5$ and $R^6$ together signify the groups $(—CH_2—)_m$, $—CH_2—O—CH_2—$,

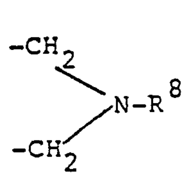

or

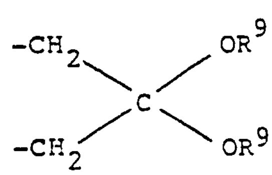

in which m represents a number 3 to 5, $R^8$ represents lower alkyl, phenyl or benzyl and $R^9$ represents lower alkyl or both $R^9$'s together represent di- or trimethylene, $R^7$ signifies methyl, lower alkoxy, di-lower alkylamino or fluorine and n signifies the number 0, 1, 2 or 3, or an optically active compound of the general formula which is present in the (R)- or (S)-form

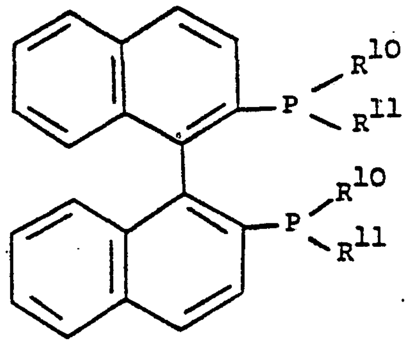

(IV)

wherein $R^{10}$ and $R^{11}$ signify phenyl or cyclohexyl and the naphthalene rings are optionally substituted in the ortho-position with methyl, ethyl, halogen, di-lower alkylamino or lower alkoxy.

2. A process in accordance with claim 1, characterized in that the isomerization is carried out by means of a compound of general formula III.

3. A process in accordance with claim 1 or 2, characterized in that as compounds of formula III there are used those in which $R^4$ signifies unsubstituted phenyl or phenyl substituted with methyl or fluorine, $R^5$ and $R^6$ are the same and signify lower alkyl or together signify the group $-CH_2-O-CH_2-$, n signifies the number 0 or 1 and $R^7$ signifies methyl, fluorine or di-lower alkylamino.

4. A process in accordance with any one of claims 1 to 3, characterized in that (R)- or (S)-(6,6'-dimethyl-2,2'-biphenylylene)-bis(diphenylphosphine) is used as the compound of formula III.

5. A process in accordance with any one of claims 1 to 4, characterized in that rhodium, iridium or cobalt, preferably rhodium, is used as the metal of Group VIII.

6. A process in accordance with any one of claims 1 to 5, characterized in that the complex of a metal of Group VIII and a compound of formula III or IV is prepared separately before the isomerization.

7. A process in accordance with any one of claims 1 to 6, characterized in that [$\eta^4$-bicyclo[2.2.1]hepta-2,5-diene][(R)- or (S)-(6,6'-dimethyl-2,2'-biphenylylene)-bis(diphenylphosphine)rhodium(I) tetrafluoroborate is used as the complex of a metal of Group VIII and an optically active compound of formula III which is present in the (R)- or (S)-form.

8. A process in accordance with any one of claims 1 to 6, characterized in that [$\eta^4$-1,5-cyclooctadiene][(R)- or (S)-(6,6'-dimethyl-2,2'-biphenylylene)-bis(diphenylphosphine)]rhodium(I) perchlorate is used as the complex of a metal of Group VIII and an optically active compound of formula III which is present in the (R)- or (S)-form.

**Revendications** (pour les Etats contractants : BE, CH, DE, FR, GB, IT, LI, NL)

1. Procédé de préparation de composés bifonctionnels, possédant une activité optique, de formule générale

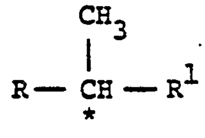

(I)

dans laquelle R représente un groupe hydroxyméthyle protégé, un groupe formyle protégé ou un groupe alcoxycarbonyle et $R^1$ représente un groupe de formule

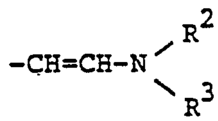

ou $-CH_2-CH=NR^2$ dans lesquelles $R^2$ et $R^3$ représentent des groupes alkyle inférieurs ou cycloalkyle ou bien $R^2$ et $R^3$ forment ensemble et avec l'atome d'azote un noyau hétérocyclique, caractérisé en ce que l'on isomérise une amine de formule générale

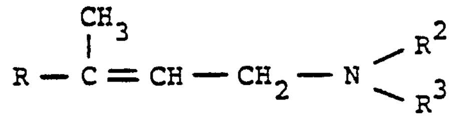

(II)

dans laquelle R, $R^2$ et $R^3$ ont les significations indiquées ci-dessus, $R^3$ pouvant en outre représenter l'hydrogène, à l'aide d'un complexe d'un métal du groupe VIII et d'un composé possédant l'activité optique, sous la forme (R) ou (S), de formule générale

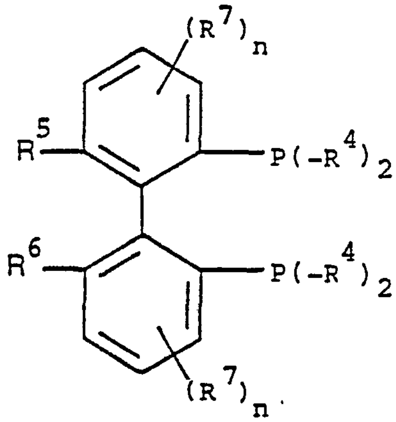

(III)

dans laquelle $R^4$ représente un groupe phényle, $R^5$ et $R^6$, ayant des significations identiques ou différentes, représentent chacun l'hydrogène, un groupe alkyle inférieur, alcoxy inférieur, di-(alkyle inférieur)-amino, hydroxyméthyle protégé, ou bien $R^5$ et $R^6$ forment ensemble les groupes $(-CH_2-)_m$, $-CH_2-O-CH_2-$,

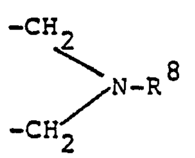

ou

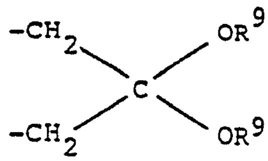

dans lesquels m est un nombre allant de 3 à 5, $R^8$ représente un groupe alkyle inférieur, phényle ou benzyle et $R^9$ représente un groupe alkyle inférieur, ou bien les deux symboles $R^9$ représentent ensemble un groupe di- ou tri-méthylène, $R^7$ représente un groupe méthyle, alcoxy inférieur, di-(alkyle inférieur)-amino ou le fluor et n est égal à 0, 1, 2 ou 3, ou d'un composé possédant l'activité optique, sous la forme (R) ou (S), de formule générale

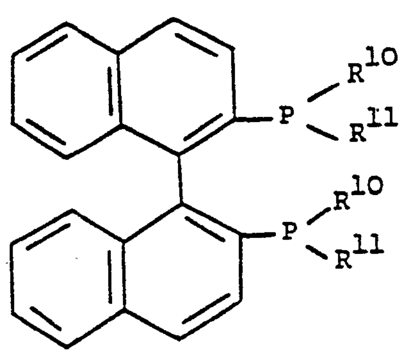

(IV)

dans laquelle $R^{10}$ et $R^{11}$ représentent des groupes phényle ou cyclohexyle et les cycles naphtaléniques sont encore substitués le cas échéant en position ortho par un groupe méthyle, éthyle, un halogène, un groupe di-(alkyle inférieur)-amino ou alcoxy inférieur.

2. Procédé selon la revendication 1, caractérisé en ce que l'isomérisation est effectuée à l'aide d'un

composé de formule générale III.

3. Procédé selon la revendication 1 ou 2, caractérisé en ce que l'on utilise des composés de formule III dans laquelle $R^4$ représente un groupe phényle non substitué ou substitué par des groupes méthyle ou le fluor, $R^5$ et $R^6$ sont identiques et représentent des groupes alkyle inférieurs ou bien, ensemble, le groupe $—CH_2—O—CH_2—$, n est égal à 0 ou 1 et $R^7$ représente un groupe méthyle, le fluor ou un groupe di-(alkyle inférieur)-amino.

4. Procédé selon l'une des revendications 1 à 3, caractérisé en ce que l'on utilise en tant que composé de formule III la (R)- ou la (S)-(6,6'-diméthyl-2,2'-biphénylylène)-bis-(diphénylphosphine).

5. Procédé selon l'une des revendications 1 à 4, caractérisé en ce que l'on utilise en tant que métal du groupe VIII le rhodium, l'iridium ou le cobalt, de préférence le rhodium.

6. Procédé selon l'une des revendications 1 à 5, caractérisé en ce que l'on prépare le complexe d'un métal du groupe VIII et d'un composé de formule III ou IV séparément, avant l'isomérisation.

7. Procédé selon l'une des revendications 1 à 6, caractérisé en ce que l'on utilise en tant que complexe d'un métal du groupe VIII et d'un composé de formule III possédant l'activité optique, sous la forme (R) ou (S), le tétrafluoroborate de [$\eta^4$-bicyclo-[2.2.1]hepta-2,5-diène][(R)- ou (S)-(6,6'-diméthyl-2,2'-biphénylylène)-bis-(diphénylphosphine)-rhodium(I).

8. Procédé selon l'une des revendications 1 à 6, caractérisé en ce que l'on utilise en tant que complexe d'un métal du groupe VIII et d'un composé de formule III possédant l'activité optique, sous la forme (R) ou (S), le perchlorate de [$\eta^4$-1,5-cyclo-octadiène][(R)- ou (S)-(6,6'-diméthyl-2,2'-biphénylylène)-bis-(diphénylphosphine)]-rhodium-(I).

9. Composés bifonctionnels, possédant une activité optique, de formule générale

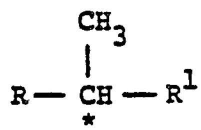

(I)

dans laquelle R représente un groupe hydroxyméthyle protégé, formyle protégé ou alcoxycarbonyle et $R^1$ représente un groupe de formule

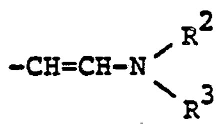

ou $—CH_2—CH=NR^2$ dans lesquelles $R^2$ et $R^3$ représentent des groupes alkyle inférieurs ou cycloalkyle ou bien $R^2$ et $R^3$ forment ensemble et avec l'atome d'azote un noyau hétérocyclique.

**Revendications** (pour l'Etat contractant AT)

1. Procédé de préparation de composés bifonctionnels, possédant une activité optique, de formule générale

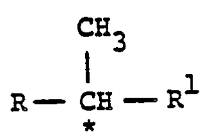

(I)

dans laquelle R représente un groupe hydroxyméthyle protégé, un groupe formyle protégé ou un groupe alcoxycarbonyle et $R^1$ représente un groupe de formule

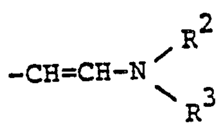

(II)

ou $—CH_2—CH=NR^2$ dans lesquelles $R^2$ et $R^3$ représentent des groupes alkyle inférieurs ou cycloalkyle ou bien $R^2$ et $R^3$ forment ensemble et avec l'atome d'azote un noyau hétérocyclique, caractérisé en ce que l'on isomérise une amine de formule générale

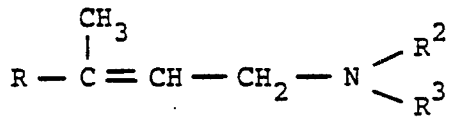

(II)

dans laquelle R, $R^2$ et $R^3$ ont les significations indiquées ci-dessus, $R^3$ pouvant en outre représenter l'hydrogène, à l'aide d'un complexe d'un métal du groupe VIII et d'un composé possédant l'activité optique, sous la forme (R) ou (S), de formule générale

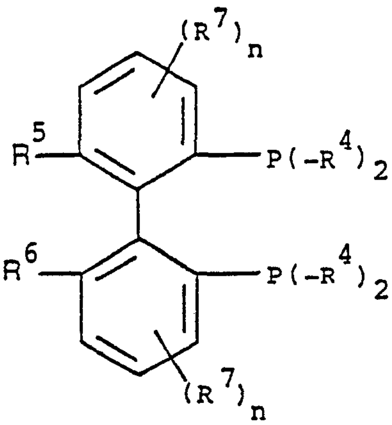

(III)

dans laquelle $R^4$ représente un groupe phényle, $R^5$ et $R^6$, ayant des significations identiques ou différentes, représentent chacun l'hydrogène, un groupe alkyle inférieur, alcoxy inférieur, di-(alkyle inférieur)-amino, hydroxyméthyle protégé, ou bien $R^5$ et $R^6$ forment ensemble les groupes $(-CH_2-)_m$, $-CH_2-O-CH_2-$,

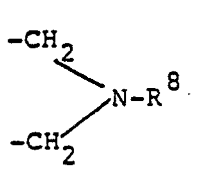 ou 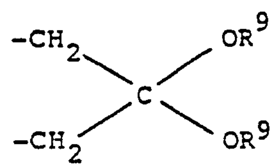

dans lesquels m est un nombre allant de 3 à 5, $R^8$ représente un groupe alkyle inférieur, phényle ou benzyle et $R^9$ représente un groupe alkyle inférieur, ou bien les deux symboles $R^9$ représentent ensemble un groupe di- ou tri-méthylène, $R^7$ représente un groupe méthyle, alcoxy inférieur, di-(alkyle inférieur)-amino ou le fluor et n est égal à 0, 1, 2 ou 3, ou d'un composé possédant l'activité optique, sous la forme (R) ou (S), de formule générale

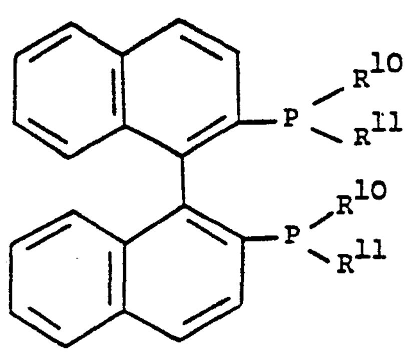

(IV)

dans laquelle $R^{10}$ et $R^{11}$ représentent des groupes phényle ou cyclohexyle et les cycles naphtaléniques sont encore substitués le cas échéant en position ortho par un groupe méthyle, éthyle, un halogène, un groupe di-(alkyle inférieur)-amino ou alcoxy inférieur.

2. Procédé selon la revendication 1, caractérisé en ce que l'isomérisation est effectuée à l'aide d'un composé de formule générale III.

3. Procédé selon la revendication 1 ou 2, caractérisé en ce que l'on utilise des composés de formule III dans laquelle $R^4$ représente un groupe phényle non substitué ou substitué par des groupes méthyle ou le fluor, $R^5$ et $R^6$ sont identiques et représentent des groupes alkyle inférieurs ou bien, ensemble, le groupe $—CH_2—O—CH_2—$, n est égal à 0 ou 1 et $R^7$ représente un groupe méthyle, le fluor ou un groupe di-(alkyle inférieur)-amino.

4. Procédé selon l'une des revendications 1 à 3, caractérisé en ce que l'on utilise en tant que composé de formule III la (R)- ou la (S)-(6,6'-diméthyl-2,2'-biphénylylène)-bis-(diphénylphosphine).

5. Procédé selon l'une des revendications 1 à 4, caractérisé en ce que l'on utilise en tant que métal du groupe VIII le rhodium, l'iridium ou le cobalt, de préférence le rhodium.

6. Procédé selon l'une des revendications 1 à 5, caractérisé en ce que l'on prépare le complexe d'un métal du groupe VIII et d'un composé de formule III ou IV séparément, avant l'isomérisation.

7. Procédé selon l'une des revendications 1 à 6, caractérisé en ce que l'on utilise en tant que complexe d'un métal du groupe VIII et d'un composé de formule III possédant l'activité optique, sous la forme (R) ou (S), le tétrafluoroborate de [$\eta^4$-bicyclo-[2.2.1]hepta-2,5-diène][(R)- ou (S)-(6,6'-diméthyl-2,2'-biphénylylène)-bis-(diphénylphosphine)-rhodium(I).

8. Procédé selon l'une des revendications 1 à 6, caractérisé en ce que l'on utilise en tant que complexe d'un métal du groupe VIII et d'un composé de formule III possédant l'activité optique, sous la forme (R) ou (S), le perchlorate de [$\eta^4$-1,5-cyclo-octadiène][(R)- ou (S)-(6,6'-diméthyl)2,2'-biphénylylène)-bis-(diphénylphosphine)]-rhodium-(I).